# EUROPEAN PATENT SPECIFICATION

(11) **EP 2 901 969 B1**
(45) Date of publication and mention of the grant of the patent: **04.07.2018**
(21) Application number: 14191052.1
(22) Date of filing: 05.03.2009
(51) Int. Cl.: A61F 2/30, A61F 2/34, A61F 2/38, A61F 2/40

(54) **METHOD OF MAKING AN EDGE-MATCHED ARTICULAR IMPLANT**
Verfahren zur Herstellung eines Gelenkimplantats mit angepasstem Rand
Procédé de fabrication d'un implant articulaire à bords concordants

(30) Priority: 05.03.2008 US 34014
(43) Date of publication of application: 05.08.2015
(62) Divisional of application: 09717686.1
(73) Proprietor: ConforMIS, Inc., Billerica, MA 01821 (US)
(72) Inventor: LANG, Philipp, Lexington, MA 02421 (US); STEINES, Daniel, Lexington, MA 02421 (US); FITZ, Wolfgang, Sherborn, MA 01770 (US)
(74) Representative: Grund, Martin

(56) References cited:
- US-A1- 2003 055 502
- US-A1- 2005 267 584

## Description

### Technical Field

The embodiments described herein relate to orthopedic methods. More particularly, methods for knee, hip, ankle, foot, shoulder, elbow, wrist and hand arthroplasty are provided.

The closest prior art is document US 2003/0055502 A1, which discloses methods for producing articular repair materials and for repairing an articular surface, including producing or employing a component with an external surface having near anatomic alignment to the surrounding structures.

### Background Art

It is common to resect the upper tibia during, for example, unicompartmental knee arthroplasty (and also bicompartmental and total knee arthroplasty), and insert a tibial implant. Typically, the tibial implant is selected from a limited, fixed number of sizes such that overhang of the implant over the resected tibial plateau is prevented. Towards this end, the tibial implant is often selected such that the implant's periphery is smaller than the tibial plateau's outer periphery. Alternatively, in select cases, the implant may overhang with the potential for interference with adjacent soft-tissues and ligaments. As a result, the implant rests on the inner cancellous bone rather than the harder, outer cortical bone located at the outer periphery of the tibial plateau. As cancellous bone is spongy, the tibial implant will tend to shift position after time.

### Summary

The present invention concerns a method of making an implant as defined in claim 1. The method may be used to make an implant configured to be fit into a cut portion of bone of a patient. The implant can include a substantially flat or substantially tray-shaped or dome-shaped body having an inferior surface, a superior surface, and an outer peripheral edge extending between the inferior and superior surface. At least a portion of the peripheral edge is configured to substantially match at least a corresponding portion of the periphery of the cut portion of the bone such that the implant is substantially supported by cortical bone.

The invention may be used to make a tibial tray configured to be fit into a tibial plateau of a patient. The tray can include a substantially flat or substantially tray-shaped or dome-shaped body having an inferior surface, a superior surface, and an outer peripheral edge extending between the inferior and superior surface. At least a portion of the peripheral edge is configured to substantially match at least a corresponding portion of the periphery of the tibial plateau when the tibial tray is implanted on the tibial plateau.

The tibial tray can include an anchor for securing the tibial tray. An entire outer portion of the peripheral edge can be configured to substantially match the corresponding periphery of the tibial plateau when the tibial tray is implanted on the tibial plateau, and an entire inner portion of the peripheral edge substantially abuts, optionally, a vertical cut in the tibial plateau. Alternatively, at least a portion of the peripheral edge can be configured to be recessed from a corresponding periphery of the tibial plateau and can be further configured to be adjacent to cortical bone when the tibial tray is implanted on the tibial plateau. In still another arrangement, at least a first outer portion of the peripheral edge can be configured to be adjacent to cortical bone and at least a second outer portion of the peripheral edge can be configured to be adjacent to cancellous bone when the tibial tray is implanted on the tibial plateau.

Additionally, the superior surface of the tray can be at least partially derived from patient-specific data of a femoral condyle. The patient-specific data can be obtained from an image of a femoral condyle, an image of a tibia or tibial plateau or a shape of an implant. The superior surface can comprise at least one curve derived from patient-specific data. The superior surface can be made of Ultra High Weight Molecular Polyethylene (UHMWPE) as well as cross-linked polyethylene.

The contour of the peripheral edge can be derived from patient-specific data, which can be derived, for example, from an image of a proximal end of a tibia. The peripheral edge of the tibial tray can be configured to rest on cortical bone when implanted on the tibial plateau. The tray can have a thickness from the inferior surface to the superior surface of about 3 to 15 mm.

In accordance with the invention, a method making an implant includes obtaining an image of at least a portion of the tibial plateau. An outer periphery of at least a portion of the tibial plateau is derived based, at least in part, on the image. An implant is provided for the tibial plateau, the implant having a periphery that includes an outer edge that substantially matches at least portions of the derived outer periphery of the tibial plateau.

In some embodiments, the periphery of the implant includes an inner edge which spans across the tibial plateau. Deriving the outer periphery of the tibial plateau may include deriving a three-dimensional representation. The image of the tibial plateau may include subchondral bone, cortical bone, normal articular cartilage and/or diseased articular cartilage. The implant may further include an anchor for securing the implant. The anchor may be a keel, peg, nub, and/or rod. The implant may include a polymer(s), a ceramic(s), a metal(s) and/or a ceramic-metal composite(s). The implant may further include an inferior surface for facing the tibial plateau, and a superior surface for facing the femur, and wherein the superior surface includes at least one of a ceramic, a metal, a polymer and a ceramic-metal composite. The implant may further include an inferior surface for facing the tibial plateau, and a superior surface for facing the femur, the method further including deriving, at least partially, the superior surface from patient-specific data of a femoral condyle. The patient specific data may be obtained from an image of a femoral condyle. The outer edge may be adapted to substantially rest on cortical bone on the tibial plateau. The implant may be secured to the tibial plateau, wherein the outer edge of the implant rests substantially on cortical bone. The implant may have a thickness of about 3 to 15 mm. At least a section of the tibial plateau may be resected.

In other embodiments, methods of making a tibial plateau implant can include obtaining an image of a knee joint including a tibial plateau. A representation of the outer tibial edge of the tibial plateau is derived from the image. An implant body is provided having a bearing surface, a tibial interface, an inner edge, and an outer edge that substantially matches the contour of the outer tibial edge.

In related embodiments, the outer edge may be adapted to substantially rest on cortical bone on the tibial plateau. The implant may be secured to the tibial plateau, wherein the outer edge of the implant rests substantially on cortical bone.

Methods of joint arthroplasty may include providing an implant for a tibial plateau having a body with a bearing surface, a tibial interface, an inner edge, and an outer edge that substantially matches the contour of the outer edge of a patient's tibial plateau. The tibial plateau can be prepared to receive the implant. The implant can be secured to the prepared implant site, wherein at least portions of the outer periphery of the tibial interface rest substantially on cortical bone.

The implant may include an anchor. The anchor may be a keel, peg, nub, and/or rod. The implant may include a bearing surface component for receiving a femoral condyle. The bearing surface component may include ceramic(s), metal(s), polymer(s) and/or ceramic-metal composite(s). The polymer may include UHMWPE. The bearing surface may be at least partially derived from patient-specific data of a tibial plateau including subchondral bone, cortical bone, normal and/or diseased cartilage, one or more femoral condyles including subchondral bone, cortical bone, normal and/or diseased cartilage. The patient-specific data may be obtained from an image of a femoral condyle or a tibial plateau or a first or an opposing second articular surface. The body may include polymer(s), ceramic(s), metal(s) and/or ceramic-metal composite(s). The contour of the outer edge may be derived from patient-specific data. The patient-specific data may be obtained from an image of a proximal tibial end. The implant may have a thickness of about 3 to 15 mm. The method may further include obtaining an image of at least a portion of the tibial plateau, and deriving the outer edge of the implant based, at least in part, on the image. The inner edge of the implant may be adapted to span across the tibial plateau. The undersurface of the implant may be flat or curved. The undersurface and/or the top surface of the implant may be at an angle other than 90 degrees relative to the sagittal or coronal axis of the tibia or the biomechanical axis.

The implant may include a body having a bearing surface, a tibial interface, an inner edge, and an outer edge that substantially matches the contour of the outer edge of a patient's tibial plateau.

Any implant, e.g. in a knee, hip, shoulder or other joint, may be made of a single material, e.g. polyethylene. The implant may also be made using two materials, e.g. a metal backing and a polyethylene insert. The polyethylene insert may be locked inside the metal backing using standard locking mechanisms as are known in the art.

The implant may include an anchor for securing the implant. The anchor may be a keel, peg, nub, and/or rod. Portions of the implants, e.g. the pegs or keel or portions of the bone facing surface may be porous coated. The undersurface of the implant may include cement pockets. The cement pockets may be open at the external margin of the implant to interface with the endosteal bone of the cut tibial plateau or the cut surface along a potential vertical cut, when used. The implant may further include a bearing surface component for receiving a femoral condyle. The bearing surface component may include a ceramic(s), metal(s), polymer(s) and/or ceramic-metal composite(s). The polymer may include UHMWPE. The bearing surface may be at least partially derived from patient-specific data of a femoral condyle or a tibial plateau or it may reflect the shape or be a mirror image of aspects of the external geometry of the femoral bearing surface, e.g. in different flexion or extension angles. The patient-specific data may be obtained from an image of a femoral condyle or a tibial plateau. The body may include polymer(s), ceramic(s), metal(s) and/or ceramic-metal composite(s). The contour of the outer edge may be derived from patient-specific data. The patient-specific data may be obtained from an image of a proximal tibial end. The outer periphery of the tibial interface may be adapted to substantially rest on cortical bone. The implant may have a thickness of about 3 to 15 mm.

The contour of the outer edge of the implant may be derived from patient-specific data. The patient-specific data may be obtained from an image of a proximal tibial end. The contour of the outer edge of the implant may be derived from an axial or near-axial cross-sectional image, a sagittal or near-sagittal cross-sectional image, a coronal or near coronal cross-sectional image, or any other cross-sectional image of the proximal tibia. Alternatively, the contour of the outer edge of the implant may be determined by creating a virtual model of the proximal tibia and performing a virtual cut on the model. The virtual cut performed on the virtual model may take into account one or more mechanical or anatomical axes of the knee.

Since the implant will be edge matched, the external contour of the implant can be convex, but it can also include concave portions. For example, in a hip joint, concave shapes can be integrated in the external contour of the device in order to achieve near 100% congruency with the shape of the acetabular rim.

### Brief Description of the Drawings

The foregoing features will be more readily understood by reference to the following detailed description, taken with reference to the accompanying drawings, in which:
**FIG. 1** is a side perspective view of a resected tibial plateau;
**FIG. 2** is a side perspective view of a tibial implant asserted on the resected tibial plateau of **FIG. 1****;**
**FIG. 3** is a diagram of a method of joint arthroplasty;
**FIG. 4** is a side perspective view of a virtual model of a proximal end of a tibia with a virtual tibial cut;
**FIG. 5** is a side perspective view of an alternate example of a tibial implant having two components asserted on two resected tibial plateaus respectively;
**FIG. 6** is a side perspective view of an alternate example of a tibial implant asserted on the resected tibial plateau of **FIG. 1****;**
**FIG. 7** is a side perspective view of an alternate example of a tibial implant asserted on the resected tibial plateau of **FIG. 1****;**
**FIG. 8** is a side perspective view of an alternate example of a tibial implant asserted on a resected tibial plateau.
**FIG. 9** is a side perspective view of an alternate example of a tibial implant asserted on a resected tibial plateau extending across the entire proximal end of the tibia.

### Detailed Description

Various methods, systems and devices for joint arthroplasty are described to provide an implant for a tibial plateau that has an outer edge that substantially matches or corresponds to the outer periphery of the tibial plateau, either the entire periphery or at least a portion of the periphery. Preferably, the outer edge of the implant rests entirely on cortical bone to provide support for the implant. However, in some embodiments, only a portion of the outer edge of the device will rest on cortical bone.

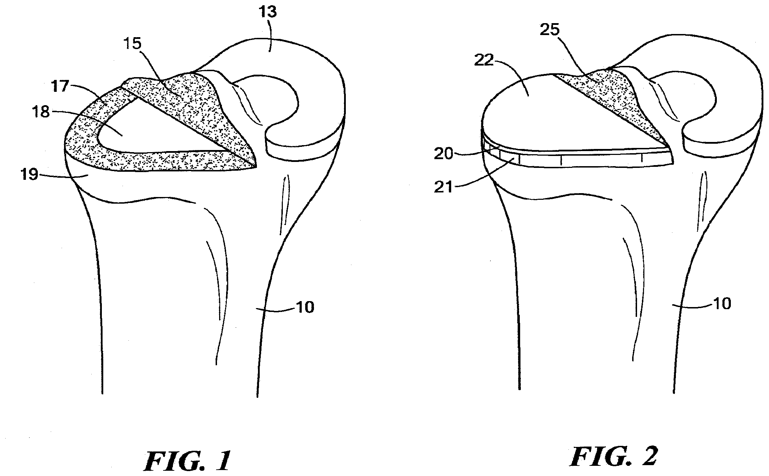

Referring to **FIG. 1****,** a resected portion of an upper (proximal) end of a tibia **10** is illustrated. The medial tibial plateau of the left tibia **10** is resected, and a lateral compartment of the tibia **10,** including a meniscus **13,** is left intact. In alternate embodiments, the lateral portion of the tibia **10** may be resected, instead, or both lateral and medial portions of the tibia **10** may be resected. Furthermore, the implant is not limited to the tibia, and may be applied to other joint surfaces where it is advantageous to have the implant rest on the outer periphery of the bone.

The resected lateral compartment of the tibia **10** may be cut, for example, along a sagittal plane to create a side wall **15.** This cut, in combination with a horizontal cut, forms a generally flat, resected tibial surface onto which a tibial implant **20** may be placed, as shown in **FIG. 2****,** described below.

As shown in **FIG. 1****,** the resected tibial surface includes an outer periphery **19** made of cortical bone **17.** Within the outer periphery of the resected tibial surface lies cancellous bone **18,** which is spongy compared to the hard cortical bone **17.**

Referring to **FIG. 2****,** an implant **20** is a tibial implant for use with a corresponding articulating femoral resurfacing or replacement implant. Implant **20** has an outer peripheral edge **21** that substantially matches the outer periphery of the tibial plateau. The matched edge ensures that the outer portion of implant **20** rests on cortical bone **17,** which provides better support than cancellous bone **18.** The outer edge **21** of the implant **20** is fully supported by the hard cortical bone **17,** as opposed to resting on the spongy cancellous bone **18.** It is to be understood that an inner peripheral edge **25** of implant **20** may span across, and may or may not contact, the tibial plateau, including cancellous bone.

The implant **20** includes an inferior surface (not shown in **FIG. 2**) that faces the tibial plateau, and a superior surface **22** that faces the femur. At least a portion of the superior surface **22** is load bearing. The superior surface **22** may be made of, for example, a ceramic, a metal, a polymer and/or a ceramic-metal composite. The inferior and superior surfaces may be derived, at least partially, from patient specific data of a femoral condyle, and/or may be matched to or defined by a curve of a corresponding femoral implant component. The patient specific data may be obtained from an image of a femoral condyle. The thickness of the implant between the superior and inferior surfaces may be about 3 to 15mm. Other structures are possible. For example, the sidewalls of a tibial tray can be rounded, tapered, recessed, proud or flush relative to the peripheral edge of the tibial plateau.

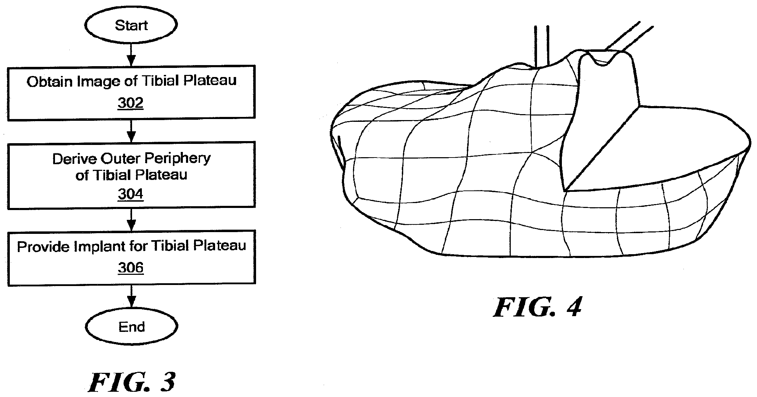

**FIG. 3** shows a method of joint arthroplasty. The method begins at step **302,** in which an image(s) of at least a portion of the tibial plateau is obtained. The obtained image may be a result of, without limitation, an MRI, CT, spiral CT, x-ray, ultrasound, digital tomosynthesis, and/or optical coherence tomography. The image of the tibial plateau may include subchondral bone, cortical bone, normal articular cartilage and/or diseased articular cartilage.

The method continues to step **304,** in which an outer periphery of at least a portion of the tibial plateau is derived, based on the image. This may be performed electronically. For example, the derivation may be performed, without limitation, by a processor (e.g., a microprocessor, microcontroller, digital signal processor, or general purpose computer), programmable logic for use with a programmable logic device (e.g., a Field Programmable Gate Array (FPGA) or other PLD), discrete components, integrated circuitry (e.g., an Application Specific Integrated Circuit (ASIC)), memory, or any other means including any combination thereof. Memory may include, for example, a diskette, a fixed disk, a Compact Disk (CD), Read Only Memory (ROM), Erasable Programmable Read-Only Memory (EPROM), and/or Random Access Memory (RAM). Computer program logic implementing all or part of the functionality previously described herein may be embodied in various forms, including, but in no way limited to, a source code form, a computer executable form, and various intermediate forms (e.g., forms generated by an assembler, compiler, linker, or locator.) Source code may include a series of computer program instructions implemented in any of various programming languages (e.g., an object code, an assembly language, or a high-level language such as Fortran, C, C++, C#, JAVA, or a scripting language) for use with various operating systems or operating environments. The source code may define and use various data structures and communication messages. The source code may be in a computer executable form (e.g., via an interpreter), or the source code may be converted (e.g., via a translator, assembler, or compiler) into a computer executable form.

Deriving the outer periphery may include deriving a cross-sectional (for example, an axial or near axial, a sagittal or near sagittal, coronal or near coronal cross-section), a two-dimensional, or a three-dimensional representation of the proximal tibia. Various scan planes may be combined to form the three dimensional representation. It may also include simulating the tibial cut on a series of two dimensional displays or on a three dimensional representation, as shown, for example, in **FIG. 4****.** The direction of the simulated tibial cut may be based on one or more mechanical or anatomical axes of the knee. These one or more axes can be derived from the same image or from one or more separate images registered into the same coordinate system as the tibial image. The height of the simulated cut may be determined from one or more reference points or landmarks on the tibia or the femur in the image. As the tibia has a taper, the outer periphery of the tibial plateau gets smaller moving in the superior to inferior direction. In preferred embodiments, the virtually derived outer periphery of the tibial plateau is thus determined at a desired cut height.

The method then continues to step **306,** in which an implant is provided for the tibial plateau. The implant has a periphery that includes an outer edge that substantially matches the derived outer periphery of the tibial plateau. In preferred embodiments, the outer edge of the tibial implant thus advantageously rests on cortical bone, as described above. The implant may be made of, without limitation, a polymer, a ceramic, a metal, and/or a ceramic metal composite.

The method may then include securing the implant to the tibial plateau. The tibial plateau may be resected, as shown for example, in **FIG. 1****.** The implant provided may include an anchor for securing the implant to the tibial plateau. The anchor may be, without limitation, a keel, peg, nub and/or rod.

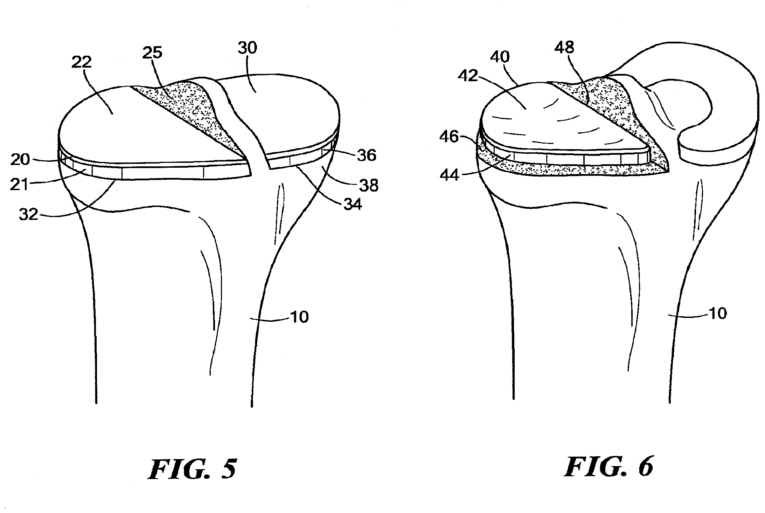

Many other alternatives are possible. Referring to **FIG. 5****,** one alternative is an implant having two separate tibial components **20** and **30.** When implanted, each component rests on a separate tibial plateau **32** and **34.** Tibial components **20** and **30** are structurally similar and made from the same materials (but could be different structures and/or materials in other embodiments). However, each is sized to correspond to match the outer periphery of the tibial plateaus **32** and **34.** Thus, tibial component **30** includes an outer sidewall **36** that substantially corresponds to an outer periphery **38** of tibial plateau **34.** Tibial components **20** and **30** could be used, for example, with a bi-compartmental resurfacing device (such as the ConforMIS iDuo), a total knee resurfacing device (such as the ConforMIS iTotal), or a total knee replacement device.

In another alternative, as shown in **FIG. 6****,** tibial tray **40** is similar in shape, structure and materials to implant **20.** A superior surface **42** of tibial tray **40** is made of UHWMPE and has a concavity in the coronal plane designed to match the curve of a corresponding femoral implant component. The curve preferably is 5 times the radius of the curve of the femoral implant in the coronal plane, but many other alternatives are possible. The outer edge **44** of tibial tray **40** does not extend completely to the outer edge **46** of the tibial plateau, but the outer edge **44** does rest on cortical bone. In still other examples, a portion of the margin of the tibial implant can be flush with the periphery of the bone while other portions are recessed from the periphery yet still lie on cortical bone. In other examples, one or more portions of the margin of the tibial implant can be flush with the periphery of the bone, one or more other portions can be recessed from the periphery yet still lie on cortical bone, and one or more other portions can be recessed from the periphery and lie over cancellous bone. In the case of the portion that lies on cancellous bone, some or all of the periphery can contact the bone or be raised up from the bone.

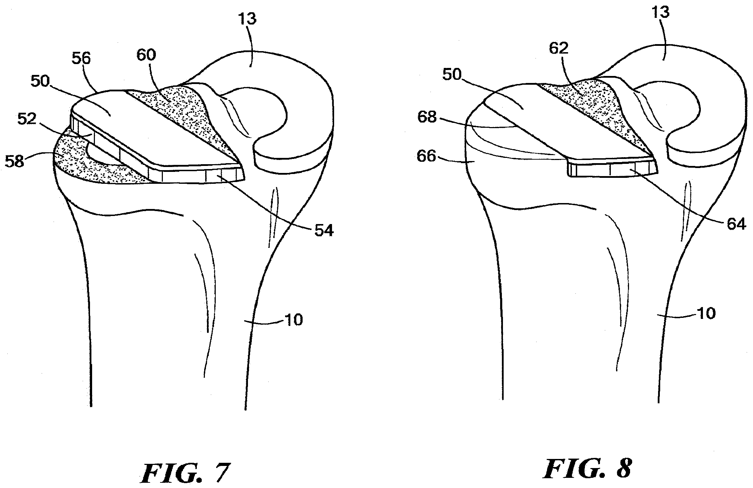

Referring to **FIG. 7****,** another example includes a narrower tibial tray **50** that has a periphery that is matched to the periphery **58** of the patient's cortical bone only at end portions **54** and **56,** while side portion **52** extends across cancellous and cortical bone. The far side of the implant **50** abuts the vertical tibial cut **60.**

Referring to **FIG. 8****,** another example includes tibial tray **50,** but tibial tray **50** is inset into an alternative tibial plateau **62** that includes a recess portion **64** and an uncut portion **66.** In this embodiment, an upper peripheral edge **68** is matched to the uncut subchondral bone, or, alternatively, uncut cartilage.

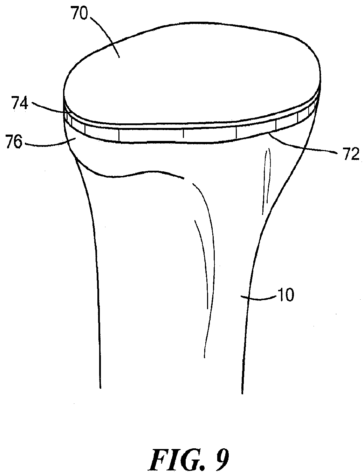

Referring to **FIG. 9****,** another example includes tibial tray **70** that rests on tibial plateau **72.** Tibial plateau **72** is cut and extends across the entire portion of the proximal end of tibia **10.** Tibial tray **70** has a peripheral side **74** that is matched to the periphery **76** of the patient's cortical bone.

Another embodiment is an implant for a shoulder joint. In a shoulder joint, the glenoid rim can substantially support the implant. The implant can be shaped or selected using an imaging test such as a CT scan or MRI scan to substantially fit onto the glenoid rim.

In another example, in a hip joint, the acetabular rim can substantially support the implant. The implant can be shaped or selected using an imaging test such as a CT scan or MRI scan to substantially fit onto the acetbular rim. The implant can be secured to the acetabular rim, for example, by forming a lip around the outer edge of the implant that rest directly on the acetabular rim or on a bone cut around the acetabular rim that exposes a flat bone surface that engages the lip of the implant when inserted. Such arrangements can provide improved structural support for implants in these joints. Such improved structural support may improve the wear and lifetime of the implants. For example, a major cause of implant failure in hip joints is loosening of the implant. Providing an improved structural support, which is advantageous

Other arrangements can apply to orthopedic implants for other joints and bones where a portion of the implant is sized to correspond to the periphery of an uncut or cut portion of bone of a patient. In such implants, the cortical bone can support most or all of the load placed on the implant at the bone-implant interface. At least a portion of the peripheral edge can be configured to substantially match at least a corresponding portion of the periphery of a cut or uncut portion of the bone such that the implant is substantially supported by cortical bone or by the rim of the articular structure. Many other alternatives are possible.

The embodiments described above are intended to be merely exemplary; other embodiments may be possible within the scope of the invention, which is defined by the appended claims.

## Claims

1. A method of making an implant (20) for repairing a joint of a patient, the method comprising:
obtaining electronic image data of the joint including at least a portion of a bone (10) associated with the joint,
simulating a cut using the electronic image data to create a simulated cut surface (15) of the bone (10) associated with the joint,
deriving an outer periphery (19) of the simulated cut surface (15); and
designing an implant having an outer periphery (21), at least a portion of which is configured to match at least a portion of the derived outer periphery (19) of the simulated cut surface (15) of the bone (10) associated with the joint and to rest on or to be adjacent to cortical bone (17) of the simulated cut surface (15) of the bone (10) associated with the joint.

2. The method of claim 1, wherein simulating the cut includes determining a height of the cut based on a reference point or a landmark of the joint of the patient;

3. The method of claim 1, further including simulating the cut at a predetermined orientation relative to one or more biomechanical or anatomical axes of the joint.

4. The method of claim 1, including determining one or more biomechanical or anatomical axes of the joint with the electronic image data and/or additional image data.

5. The method of any of the previous claims, wherein the joint of the patient is a knee, a hip, an ankle, a joint of the foot, a shoulder, an elbow, a wrist or a joint of the hand.

6. The method of any of the previous claims, wherein the joint is a knee.

## Patentansprüche

1. Ein Verfahren zur Herstellung eines Implantats (20), um das Gelenk eines Patienten zu reparieren, wobei das Verfahren umfasst:
das Einholen elektronischer Bilddaten des Gelenks einschließlich mindestens eines Teils eines Knochens (10), der mit dem Gelenk assoziiert ist,
das Simulieren eines Schnittes unter Verwendung der elektronischen Bilddaten, um eine simulierte Schnittoberfläche (15) des Knochens (10), der mit dem Gelenk assoziiert ist, zu erstellen,
die Ableitung eines äußeren Randes (19) der simulierten Schnittoberfläche (15); und
das Design eines Implantats, das einen äußeren Rand (21) hat, von dem mindestens ein Teil so konfiguriert ist, dass er mit mindestens einem Teil des abgeleiteten äußeren Randes (19) der simulierten Schnittoberfläche (15) des Knochens (10), der mit dem Gelenk assoziiert ist, übereinstimmt und auf oder neben dem kortikalen Knochen (17) der simulierten Schnittoberfläche (15) des Knochens (10), der mit dem Gelenk assoziiert ist, liegt.

2. Das Verfahren nach Anspruch 1, worin die Simulation des Schnittes die Bestimmung einer Schnitttiefe basierend auf einem Referenzpunkt oder einer Landmarke des Gelenks des Patienten, einschließt.

3. Das Verfahren nach Anspruch 1, ferner umfassend die Simulation des Schnittes in einer zuvor bestimmten Richtung relativ zu einer oder mehr biomechanischer oder anatomischer Achsen des Gelenks.

4. Das Verfahren nach Anspruch 1, einschließlich der Bestimmung einer oder mehr biomechanischer oder anatomischer Achsen des Gelenks mit den elektronischen Bilddaten und/oder zusätzlichen Bilddaten.

5. Das Verfahren eines der vorherigen Ansprüche, worin das Gelenk des Patienten ein Knie, eine Hüfte, ein Knöchel, ein Gelenk des Fußes, eine Schulter, ein Ellbogen, ein Handgelenk oder ein Gelenk der Hand ist.

6. Das Verfahren eines der vorherigen Ansprüche, worin das Gelenk ein Knie ist.

## Revendications

1. Procédé de fabrication d'un implant (20) pour réparer une articulation d'un patient, le procédé comprenant :
l'obtention de données d'image électronique de l'articulation comprenant au moins une partie d'un os (10) associé à l'articulation,
la simulation d'une découpe en utilisant les données d'image électronique pour créer une surface de découpe simulée (15) de l'os (10) associé à l'articulation,
la déduction d'une périphérie extérieure (19) de la surface de découpe simulée (15) ; et
la conception d'un implant ayant une périphérie extérieure (21), dont au moins une partie est configurée pour correspondre à au moins une partie de la périphérie extérieure déduite (19) de la surface de découpe simulée (15) de l'os (10) associé à l'articulation et pour reposer sur ou être adjacent à l'os cortical (17) de la surface de découpe simulée (15) de l'os (10) associé à l'articulation.

2. Procédé selon la revendication 1, dans lequel la simulation de la découpe comprend la détermination d'une hauteur de la découpe sur la base d'un point de référence ou d'un point de repère de l'articulation du patient.

3. Procédé selon la revendication 1, comprenant en outre la simulation de la découpe à une orientation prédéterminée relativement à un ou plusieurs axes biomécaniques ou anatomiques de l'articulation.

4. Procédé selon la revendication 1, comprenant la détermination d'un ou plusieurs axes biomécaniques ou anatomiques de l'articulation avec les données d'image électronique et/ou des données d'image supplémentaires.

5. Procédé selon l'une quelconque des revendications précédentes, dans lequel l'articulation du patient est un genou, une hanche, une cheville, une articulation du pied, une épaule, un coude, un poignet ou une articulation de la main.

6. Procédé selon l'une quelconque des revendications précédentes, dans lequel l'articulation est un genou.
